# EUROPEAN PATENT APPLICATION

(11) **EP 2 833 122 A1**
(43) Date of publication of application: **04.02.2015**
(21) Application number: 13767761.3
(22) Date of filing: 29.03.2013
(51) Int. Cl.: G01N 21/47

(54) **OIL FILM DETECTION DEVICE**

(30) Priority: 30.03.2012 JP 2012080955; 30.03.2012 JP 2012081091
(71) Applicant: DKK-Toa Corporation, Saitama 350-1388 (JP)
(72) Inventor: OKUMURA Taketo, Shinjuku-ku, Tokyo 169-8648 (JP); ISHITOBI Tsuyoshi, Shinjuku-ku, Tokyo 169-8648 (JP)
(74) Representative: Schmidbauer, Andreas Konrad
(86) International application number: PCT/JP2013/059525
(87) International publication number: WO 2013/147158

(57) **Abstract**

Provided is an oil film detection device capable of detecting an oil film reliably and accurately even when separated by a distance from a water surface (detection target surface).

Provided is an oil film detection device including, in a detector W, an accommodating portion 5 which accommodates at least a light source 1, a concave mirror 2, and a light-receiving portion 4 , and a window 6 which closes the accommodating portion 5 in a watertight manner, for detecting an oil film existing on a detection target surface owing to that light is radiated from the light source 1 toward a detection target surface, reflection light from the detection target surface is collected by the concave mirror 2 and received by the light-receiving portion 4. Here, the light source 1 is arranged above the concave mirror and emits light approximately in the vertical direction. The concave mirror 2 is arranged so that an optical axis of the concave mirror 2 is perpendicular to the horizontal direction and has a through-hole 3, as a position deviated from the optical axis of the concave mirror 2, through which emitted light from the light source 1 passes. The light-receiving portion 4 is arranged at a focal position on the optical axis of the concave mirror 2.

## Description

### TECHNICAL FIELD

The present invention relates to an oil film detection device for detecting an oil film existing on a water surface (detection target surface) of a water purification plant, a river, a lake, or the like.

### BACKGROUND ART

For example, when an oil film exists at a water purification plant, a river, a lake, or the like, it may be required to take measures such as water withdrawal stopping. When an oil film exists at factory effluent, it may be required to take measures such as checking of processing steps and stopping of water discharge. Therefore, oil film detection on a water surface has been performed conventionally. A variety of types thereof have been known as an oil film detection device. There has been known an oil film detection device which detects an oil film while measuring intensity of reflection light from a water surface as focusing on that a reflection rate of light from an oil film is higher than a reflection rate of light from a water surface.

For example, Patent Document 1 discloses an oil film detection device which detects an oil film existing on a detection target surface by radiating laser light from a light source to the detection target surface such as a water surface and receiving reflection light from the detection target surface. Here, the oil film detection device includes a two-dimensional scanning portion which performs scanning with laser light two-dimensionally with displacement generated by applying a drive voltage in X direction and a drive voltage in Y direction respectively and forms a desired plane as a radiation range of laser light on the detection target surface.

Further, Patent Document 2 discloses an oil film detection device including a light-emitting portion which emits light toward a water surface and a light-receiving portion which receives reflection light from the water surface and detecting presence or absence of an oil film on the water surface based on an amount of reflection light received by the light-receiving portion. Here, the oil film detection device includes a concave lens which causes light emitted from the light-emitting portion to diverge and radiates the light as a diverging beam to the water surface, a recurrent reflector which is arranged between the concave lens and the water surface and, when reflection light of the diverging beam from the water surface enters, which reflects the incoming light toward the water surface on a path being approximately the same as the incoming path. The light reflected from the reflector penetrates the concave lens as a convergent beam after being reflected from the water surface and is received by the light-receiving portion.

There have been known a floating type and a fixed type for an oil film detection device. With a floating type, since an oil film detection device is used for measurement as being floated at a water surface, it is possible to continuously follow variation of water level. In contrast, with a fixed type as disclosed in Patent Document 1 and Patent Document 2, a distance between a water surface and a detector varies in accordance with water level variation. In view of the above, a problem caused by a positional relation of an optical system varying in accordance with water level variation is solved owing to such that light is radiated continuously to a desired range of a water surface (detection target surface) with a structure of radiating light approximately in the vertical direction toward the water surface. However, when a water surface is raised abnormally due to heavy rain or the like, there is a fear that a detector installed at a position relatively close to a normal water surface is submerged. Accordingly, it has been desired to provide an oil film detection device capable of accurately detecting an oil film as well as measuring even when a detector is installed as being separated by a sufficient distance from a water surface.

### CITED DOCUMENT

### PATENT DOCUMENT

Patent Document 1: International Publication WO2009/022649
Patent Document 2: Japanese Patent Application Laid-open No. 2005-24414

### SUMMARY OF THE INVENTION

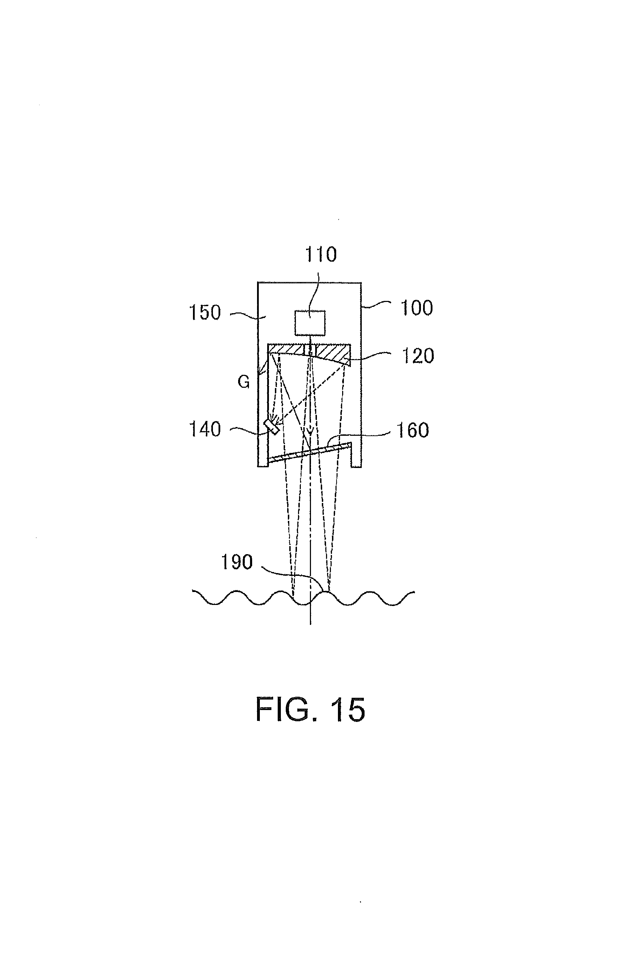

For example, a detector of a conventional oil film detection device as disclosed in Patent Document 1 has a structure, for dew condensation prevention of an optical system and protection of electric parts, that an accommodating portion 150 accommodates a light-source and scanning portion 110, a concave mirror 120, a light-receiving portion 140, and the like and is closed by a window 160 in a watertight manner, as illustrated in FIG. 15. Here, the window 160 of a detector 100 is attached to the detector 100 as being inclined from the horizontal plane by a predetermined angle. This is to prevent light emitted from the light source 110 from becoming to stray light after reflected from each of the top face and the bottom face of the window 160. For example, even when light emitted from the light source 110 and reflected from the top face of the window 160 enters to the concave mirror 120, the reflection light is introduced to position G and is prevented from entering to the light-receiving portion 140. However, regarding the structure to close the accommodating portion 150 in a watertight manner with the window 160 which is inclined from the horizontal plane, designing and processing are complicated and workability of assembling is inferior. Accordingly, there has been a problem of cost increase.

Further, in a conventional oil film detection device, a so-called off-axis parabolic mirror or the like whose focal position is shifted as concave mirrors 120, 40, as illustrated, for example, in FIG. 15, FIGs 6 and 8 of Patent Document 1, and the like is adopted so that light-collecting can be effectively performed even with small area while preventing reflection light from a detection target surface from being blocked by a light-receiving portion. Here, to detect an oil film more accurately, the concave mirror is required to be enlarged. Since a large block is required to be machined for manufacturing a large off-axis parabolic mirror, there has been a problem of cost increase. Further, since opening size of the detector is enlarged as well when the concave mirror is enlarged, there has been a problem that a dimension of the detector in the vertical direction becomes large and the detector is upsized when the window inclined as described above is assembled therein.

Meanwhile, for example, a conventional oil film detection device as disclosed in Patent Document 2 performs detection by causing light to reciprocate while reflection light from a water surface is reflected by a recurrent reflector. Accordingly, there has been a problem that measurement cannot be performed sensitively because a light amount arriving at the light-receiving portion becomes significantly small when separated by a distance from the water surface due to large attenuation of reflection light.

Further, for example, in a conventional oil film detection device as illustrated in FIG. 15, it is required to finely adjust a sensor position in accordance with a positional relation of an optical system.

Here, there has been a problem that a mechanism to perform positional adjustment while preventing reflection light from a water surface from being blocked.

To solve the abovementioned problems, an object of the present invention is to provide an oil film detection device capable of detecting an oil film reliably and accurately even when separated by a distance from a water surface (detection target surface).

To achieve the abovementioned object, the present invention provides an oil film detection device, comprising, in a detector which is arranged approximately in parallel to a detection target surface extended in the horizontal direction, an accommodating portion which accommodates at least a light source, a concave mirror, and a light-receiving portion, and a window which closes the accommodating portion in a watertight manner, for detecting an oil film existing on the detection target surface owing to that light is radiated from the light source toward the detection target surface, and reflection light from the detection target surface is collected by the concave mirror and received by the light-receiving portion. Here, the light source is arranged above the concave mirror and emitting light approximately in the vertical direction, the concave mirror is arranged so that an optical axis of the concave mirror is perpendicular to the horizontal direction and having a through-hole, at a position deviated from the optical axis of the concave mirror, through which emitted light from the light source passes, and the light-receiving portion is arranged at a focal position on the optical axis of the concave mirror.

Further, the window is arranged in parallel to a horizontal section of the detector, and an emitting window through which the emitted light penetrates is arranged at a part of the window as being inclined from the horizontal surface by a predetermined angle.

Further, the light-receiving portion is held by at least two thin plates, each thin plate having a width in a direction of an optical axis of radiation from the light source, and a length larger than the width and a thickness smaller than the width respectively in a direction perpendicular to the optical axis, an approximate-T-shaped cutout capable of allowing a thin-plate-use male screw having a head portion to be inserted thereto from a direction of the thickness is formed at each thin plate on a plane extending in a direction of the width and a direction of the length, the detector includes support pillars corresponding to the respective thin plates, the support pillar is formed with a slit longer than the width of the thin plate in the longitudinal direction and a female thread formed in the slit to allow the thin-plate-use male screw to be screwed thereto, and the thin plates are anchored to the corresponding support pillars in a positionally adjustable manner owing to that each thin plate is inserted to the slit of the corresponding support pillar and the thin-plate-use male screw is screwed to the female thread in the slit so that a seating face thereof pushes a thickness section of the approximate-T-shaped cutout in a direction toward the support pillar.

In the present invention, light is radiated from the light source toward the detection target surface approximately in the vertical direction, the concave mirror which collects light reflected from the detection target surface is arranged so that the optical axis thereof is perpendicular to the horizontal direction, and the light-receiving portion is arranged at the focal position on the optical axis of the concave mirror (i.e., a concave mirror not being an off-axis concave mirror is adopted). Accordingly, an oil film can be detected reliably and accurately even when separated by a distance from the water surface (detection target surface). Further, the window which closes the accommodating portion in the detector in a watertight manner is arranged in parallel to the horizontal section of the detector. Accordingly, it is possible to adopt a general structure to close the accommodating portion in a watertight manner, providing easy machining and excellent assembling workability. Further, the emitting window through which emitted light penetrates is arranged at a part of the window as being inclined from the horizontal plane by the predetermined angle. Accordingly, it is possible to provide an oil film detection device capable of accurately detecting an oil film while avoiding upsizing of the detector.

Further, the light-receiving portion is held by the thin plate and can be anchored while easily performing positional adjustment using the thin-plate-use male screw when the thin plate is anchored to the support pillar. Accordingly, positional adjustment of the light-receiving portion can be easily performed while preventing reflection light from the water surface from being blocked.

### BRIEF DESCRIPTION OF THE DRAWINGS

FIG. 1 is a schematic sectional view schematically illustrating a structural example and an operation of a detector of an oil film detection device in a first embodiment of the present invention.
FIG. 2 is a bottom view viewing the detector of the oil film detection device illustrated in FIG. 1 from a lower face (water surface) side as illustrating a positional relation between a light-receiving portion and an emitting window.
FIG. 3 is a sectional view at A-A of the detector of the oil film detection device illustrated in FIG. 1.
FIG. 4 illustrates an example of a schematic view of a thin plate in a second embodiment of the present invention.
FIG. 5 is a schematic view of a thin-plate-use male screw in the second embodiment of the present invention.
FIG. 6 is a schematic view of a support pillar in the second embodiment of the present invention.
FIG. 7 is a view illustrating a state that the thin-plate-use male screw illustrated in FIG. 5 is inserted from a thickness direction to an approximate-T-shaped cutout of the thin plate illustrated in FIG. 4.
FIG. 8 is a view illustrating a state that the thin plate is inserted to a slit of the support pillar and anchored thereto in the second embodiment of the present invention.
FIG. 9 is a schematic view of an anchoring screw in a third embodiment of the present invention.
FIG. 10 is a schematic view of a support pillar of the third embodiment of the present invention.
FIG. 11 is a view illustrating a state that the thin plate is inserted to a slit of the support pillar and anchored thereto in the third embodiment of the present invention.
FIG. 12 is a schematic view of another example of the thin plate in the second and third embodiments of the present invention.
FIG. 13 is a view illustrating a state that the thin-plate-use male screw illustrated in FIG. 5 is inserted from a thickness direction to an approximate-T-shaped cutout of the thin plate illustrated in FIG. 12.
FIG. 14 is a view illustrating a state that the thin plate illustrated in FIG. 12 is inserted to the slit of the support pillar and anchored thereto in the second and third embodiments of the present invention.
FIG. 15 is a schematic sectional view schematically illustrating a structural example and an operation of a detector of a conventional oil film detection device.

### EMBODIMENTS OF THE INVENTION

In the following, embodiments of the present invention will be described in detail with reference to the drawings.

### First embodiment

An oil film detection device according to the present invention adopts an optical type (light reflection method) using a laser light source. The method utilizes a difference between optical reflection rates at a water surface and an oil film. In general, it is known that an optical reflection rate at a water surface is about 2% and an optical reflection rate at an oil film fluctuates between 3% and 4%. Thus, intensity of reflection light from an oil film is one and half to two times as high as that from a water surface. Accordingly, owing to that light having constant intensity is radiated to a water surface, intensity of reflection light (light-receiving amount) is measured by a detector, and a variety of arithmetic processes are performed by a converter, presence or absence of an oil film can be detected and determined. An alarm is output when an oil film is detected. Subsequently, it is determined to stop water withdrawal, for example.

Here, in addition to an advantage of not being a contact type such as a fiber type and an electrostatic type, that is, being a non-contact type, such an optical-type oil film detection device using a laser light source has an advantage that reflection light can be detected even when separated by a distance from a water surface due to higher intensity and superior linearity compared to other light sources (e.g., an LED light source and the like).

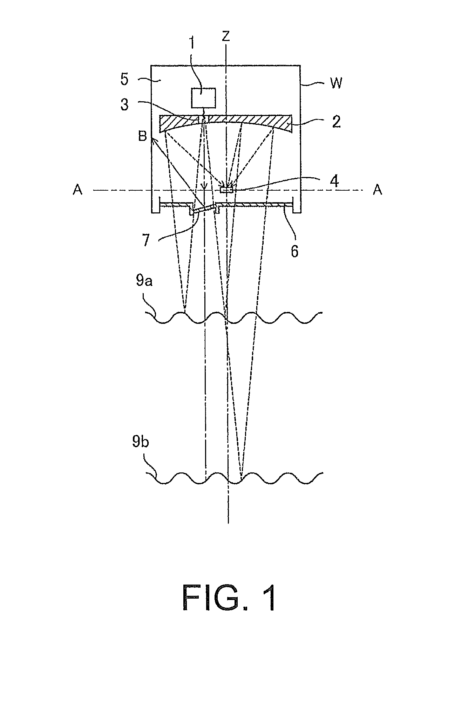

FIG. 1 is a schematic sectional view schematically illustrating a structural example and operation of a detector of an oil film detection device in a first embodiment of the present invention. The oil film detection device according to the first embodiment includes a detector W illustrated in FIG. 1 and a converter (not illustrated). The detector W and the converter are connected by a cable, so that a signal based on intensity (light-receiving amount) measured by the detector W is transmitted to the converter. The converter is capable of detecting and determining presence or absence of an oil film as performing a variety of arithmetic processes, and outputting an alarm and displaying a state thereof on a display portion when an oil film is detected. Further, the converter is capable of outputting a control signal to control the detector W.

The detector W includes a light-source and scanning portion 1, an accommodating portion 5 which accommodates a concave mirror 2 and a light-receiving portion 4, and a window 6 which closes the accommodating portion 5 in a watertight manner. The detector W is arranged above a water surface 9a, 9b being a detection target surface to be approximately in parallel to the water surface 9a, 9b (approximately in parallel to the horizontal direction). Here, a chassis of the detector W is formed of a non-transmissive material. Accordingly, light does not enter to the inside of the accommodating portion 5 through a portion other than the window 6 (including an emitting window 7).

The light-source and scanning portion 1 includes a laser light source and a scanning portion to scan with the light source. Owing to scanning with laser light, a desired plane is formed as a laser light radiation range on a detection target surface. A vibration element and the like may be used as a structure for scanning with laser light. Here, since a scanning mechanism is a known technology, detailed description is skipped. The light-source and scanning portion 1 is arranged above the concave mirror 2 so that emitted laser light is radiated toward the water surface (detection target surface) 9a, 9b approximately in the vertical direction.

Here, the oil film detection device in the first embodiment adopts the concave mirror 2 whose focal position where light collected by a reflection face thereof is focused is on the center axis of the reflection face. In other words, the center axis is matched with an optical axis Z, that is, the concave mirror 2 is not a so-called off-axis concave mirror. Further, the concave mirror 2 is arranged so that the optical axis Z is perpendicular to the horizontal direction. The concave mirror 2 includes a through-hole 3 at a position deviated from the optical axis Z. Here, light emitted from the light-source and scanning portion 1, passing through the through-hole 3, and reflected from the water surface 9a, 9b can be entered into the light-receiving portion 4 which is arranged at the focal position on the optical axis Z (approximately on the center axis of the detector W) as being collected by the reflection face. The concave mirror 2 is simply required not to be a so-called off-axis concave mirror. A parabolic mirror, an ellipsoidal mirror, or the like which is normally used may be adopted as the concave mirror 2. Further, it is preferable that the through-hole 3 is the same in size as or slightly larger than the range through which light emitted from the light-source and scanning portion 1 for scanning passes. From a viewpoint of effective detection of reflection light from the water surface 9a, 9b, it is preferable that the through-hole 3 is located closer to the optical axis Z of the concave mirror 2 (center part of the concave mirror 2). However, the through-hole 3 is required to be located so that light passing through the through-hole 3 does not enter directly to the light-receiving portion 4.

The window 6 of the oil film detection device in the first embodiment is arranged to close the accommodating portion 5 in the detector W in a watertight manner in parallel to a horizontal section of the detector W. Further, the window 6 includes, at a part thereof, the emitting window 7 which is inclined from the horizontal plane by a predetermined angle.

The emitting window 7 is located on the vertical direction from the light-source and scanning portion 1 enabling light emitted from the light source to penetrate therethrough. The inclination angle of the emitting window 7 may be determined in accordance with curvature of the concave mirror 2, size of the light-receiving portion 4, location of the emitting window 7, and the like under any of conditions that light emitted from the light source and reflected from each of a top face and a bottom face of the emitting window 7 (1) is to be introduced to a position (e.g., position B) deviated from the concave mirror 2 and (2) is prevented from entering into the light-receiving portion 4 and outgoing to the outside of the detector W via the window 6 (including the emitting window 7) even when incoming to the concave mirror 2 and reflected further. Further, it is preferable that the emitting window 7 is the same in size as or slightly larger than the range through which light emitted from the light-source and scanning portion 1 for scanning penetrates.

Here, the window 6 and the emitting window 7 are simply required to enable light emitted from the light source and reflection light to penetrate therethrough. From a viewpoint of easiness of machining, weight, cost, and the like, transparent resin such as acrylic is preferably used for forming. However, without limitation for material, it is also possible to use glass or the like.

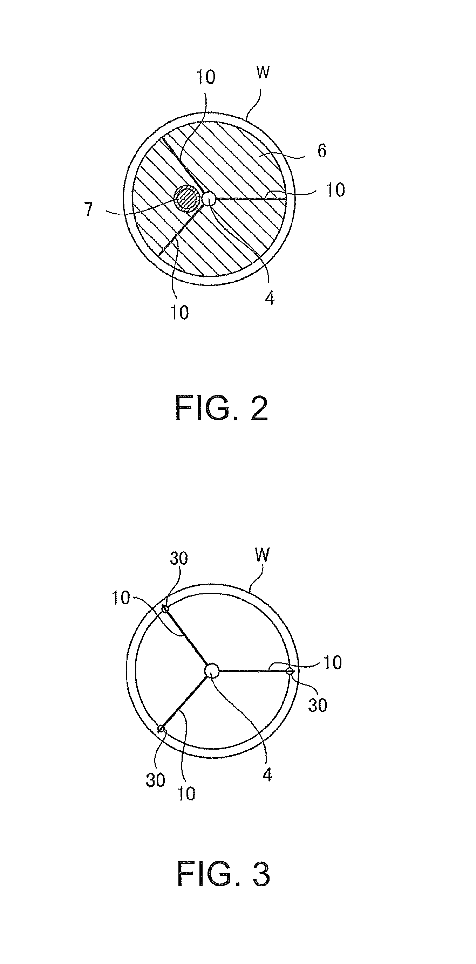

FIG. 2 is a bottom view viewing the detector of the oil film detection device illustrated in FIG. 1 from a lower face (water surface) side as illustrating a positional relation between the light-receiving portion 4 and the emitting window 7. As described above, although the through-hole 3 is preferably located closer to the optical axis Z of the concave mirror 2 (center part of the concave mirror 2), the through-hole 3 is required to be located to prevent light passing through the through-hole 3 from entering directly to the light-receiving portion 4. Accordingly, the emitting window 7 is also arranged at a position without overlapping to the light-receiving portion 4, so that light proceeding toward the emitting window 7 is prevented from being blocked by the light-receiving portion 4. That is, the light-source and scanning portion 1, the through hole 3, and the emitting window 7 are arranged in parallel to the optical axis at positions slightly deviated from the optical axis Z (center part of the concave mirror 2).

Here, a thin plate 10 is attached to the detector W for anchoring the light-receiving portion 4 at the focal position on the optical axis Z of the concave mirror 2, so that the position of the light-receiving portion 4 can be finely adjusted. In FIG. 1, the thin plate 10 is not illustrated.

Next, action and operation of the oil film detection device in the present embodiment will be described with reference to FIG. 1.

Light emitted from the light-source and scanning portion 1 for scanning is radiated approximately in the vertical direction in a range having spread as illustrated by broken lines. The light is to penetrate through the emitting window 7 arranged at a part of the window 6. Since the emitting window 7 is inclined by the predetermined angle, light reflected from each of the top face and the bottom face thereof is to be introduced to the position B deviated from the concave mirror 2 so as not to be stray light which causes interference in oil film detection.

Light penetrating through the emitting window 7 is reflected from the water surface 9a, 9b and arrives at the light-receiving portion 4 via the concave mirror 2 after penetrating through the window 6. The broken lines in FIG. 1 illustrates an optical path at an outer border of the light radiation range in a case that the water surface 9a, 9b is assumed to be a perfect plane. In actual measurement, since fluctuations (waves) exist on the water surface 9a, 9b, reflection light does not necessarily return to the concave mirror 2 continuously on the constant optical path. The possibility to detect an oil film is increased with increase of area of the reflection face of the concave mirror 2.

The water surface 9a is in a state that water level is high and the water surface 9b is in a state that water level is low. Since light emitted from the light source and scanning portion 1 is radiated approximately in the vertical direction, a plane to which the light is radiated is continuously formed on the water surface 9a, 9b and light reflected from the surface can be caught by the concave mirror 2.

In a case that a parabolic mirror is used as the concave mirror 2, all light in parallel to the optical axis is collected to the focal point. Here, when the light-receiving portion 4 has a certain degree of area (size of a light-receiving face), it is possible to collect reflection light which is not in parallel thereto perfectly.

A signal based on intensity of light (light-receiving amount) detected by the light-receiving portion 4 is transmitted to the converter to have a variety of arithmetic processes performed thereon and is used for determination of presence or absence of an oil film. When an oil film is detected, a process such as outputting an alarm signal is performed.

Thus, owing to that the light-source and scanning portion 1, the through-hole 3, and the emitting window 7 are arranged in parallel to the optical axis at positions deviated from the center of the concave mirror 2, laser light emitted from the light-source and scanning portion 1 is prevented from being blocked by the light-receiving portion 4 even though the light-receiving portion 4 is arranged at the focal position on the optical axis Z of the concave mirror 2. Accordingly, reflection light from the water surface (detection target surface) 9a, 9b can be effectively collected.

Further, since light from the light-source and scanning portion 1 is emitted approximately in the vertical direction, the light can be reliably radiated to the detection target surface and reflection light from the detection target surface can be reliably collected even when a distance between the detector W and the detection target surface is varied, that is, even when water surface height is varied (see the water surface 9a and the water surface 9b in FIG. 1).

Further, in the oil film detection device according to the first embodiment of the present invention, the window 6 which closes the accommodating portion 5 in the detector W in a watertight manner is arranged in parallel to the horizontal section of the detector W. Accordingly, it is possible to adopt a general structure to close the accommodating portion 5 in a watertight manner, providing easy machining and excellent assembling workability.

To prevent laser light emitted from the light-source and scanning portion 1 from being detected by the light-receiving portion 4 as stray light after reflected from each of the top face and the bottom face of the window 6, the emitting window 7 is arranged as being inclined from the optical axis, that is, inclined from the horizontal plane by the predetermined angle. Here, assuming that a single window is obliquely attached to the oil film detection device in the first embodiment illustrated in FIG. 1 as being similar to the conventional oil film detection device illustrated in FIG. 15, the device becomes significantly large in the vertical direction. Therefore, in the first embodiment of the present invention, only the emitting window 7 through which laser light emitted from the light-source and scanning portion 1 penetrates is arranged as being inclined from the optical axis (inclined from the horizontal plane by the predetermined angle) while the window 6 is arranged in parallel to the horizontal section of the detector W.

As a result, in the oil film detection device according to the present embodiment of the present invention similarly to the conventional oil film detection device illustrated in FIG. 15, laser light emitted from the light-source and scanning portion 1 is prevented from becoming to stray light after reflected from the window 6. Accordingly, oil film detection can be accurately performed.

In the prior art, the light-receiving portion is required to be arranged at an end of the detector. Therefore, positional adjustment of the light-receiving portion for effective light collection is a troublesome task. However, since light is collected toward the center in a case of using the concave mirror 2 not being a so-called off-axis concave mirror, light collection can be performed consequently and effectively simply by arranging the light-receiving portion 4 at the center. In addition, there is an advantage that the concave mirror 2 is easy to manufacture at low cost compared to a so-called off-axis concave mirror (concave mirror 120).

Further, the window 6 which closes the accommodating portion 5 in the detector W is arranged in parallel to the horizontal section of the detector W and only a part thereof through which laser light emitted from the light-source and scanning portion 1 penetrates is formed as the emitting window 7 which is inclined from the optical axis. Accordingly, even when a concave mirror which is larger than a conventional concave mirror is used, the size in the height direction can be kept nearly equal to the size in the prior art, providing superior space efficiency.

As described above, according to the oil film detection device according to the first embodiment of the present invention, measurement accuracy and assembling workability can be improved while avoiding cost increase and upsizing. Assuming that oil film detection can be reliably performed only under the condition that a distance to the water surface (detection target surface) 9a is shorter than, for example, five meters or so in the conventional oil film detection device, oil film detection can be performed even when a distance to the water surface (detection target surface) 9b is, for example, ten meters or so in the oil film detection device according to the present embodiment.

### Second embodiment

FIG. 3 is a sectional view at A-A of the detector of the oil film detection device illustrated in FIG. 1. FIG. 3 illustrates a state that the light-receiving portion 4 is anchored to a support pillar 30 of the detector W via the thin plate 10 for anchoring the light-receiving portion 4 approximately on the center axis (optical axis Z) of the detector W.

Here, the thin plate 10 for anchoring the light-receiving portion 4 and the support pillar 30 of the detector W are not illustrated in FIG. 1.

Here, to prevent to the extent possible the thin plate 10 for anchoring the light-receiving portion 4 from blocking laser light emitted from the light-source and scanning portion 1 and reflection light reflected from the water surface (detection target surface) 9a, 9b, it is desirable that thickness of the thin plate 10 is small to the extent possible. However, in a device having a normal size as the oil film detection device illustrated in FIG. 1, the thin plate 10 is required to have a certain level of strength because the receiving portion 4 and the thin plate 10 are to have a certain level of size and weight.

The light-receiving portion 4 is required to be arranged at the focal position on the optical axis Z of the concave mirror 2 (approximately on the center axis of the detector W). Here, fine adjustment is required in accordance with individual difference and mounting state of each device.

Consequently, the thin plate 10 is required to have a sectional shape being thin to the extent possible (thin plate) and to include a mechanism capable of performing positional adjustment of the light-receiving portion 4.

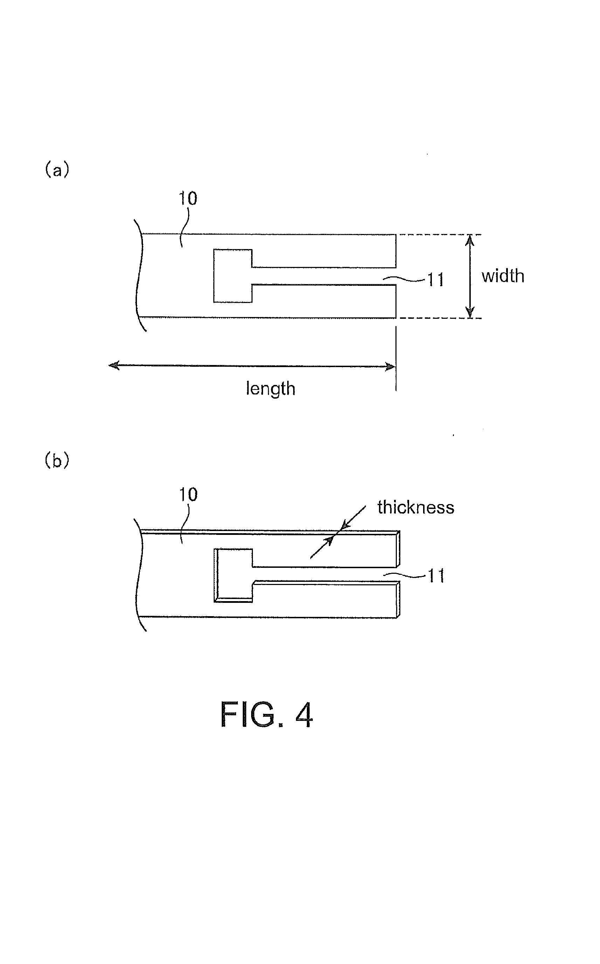

In the oil film detection device according to a second embodiment, as illustrated in FIG. 3, the light-receiving portion 4 is anchored by three thin plates 10. Here, three support pillars 30 are arranged as well corresponding to the respective thin plates 10. As described below, a thin plate anchoring method as illustrated in FIGs. 4 to 8 is adopted as a method to anchor the respective thin plates 10 to the corresponding support pillars 30.

As illustrated in FIG. 4, the thin plate 10 is arranged to have a width in a direction of the optical axis of radiation from the light-source and scanning portion 1, a length larger than the width in a direction connecting the light-receiving portion 4 and the support pillar 30 of the detector W among directions perpendicular to the optical axis, and a thickness smaller than the width in the other direction perpendicular to the optical axis.

One end (not illustrated) of the thin plate 10 in the length direction is connected to the light-receiving portion 4 and the other end thereof is anchored to the support pillar 30 of the detector W. FIG. 4(a) is a plane view of the other end side (the side to be anchored to the support pillar 30 of the detector W) on a plane extending in the width direction and the length direction and FIG. 4(b) is a perspective view thereof.

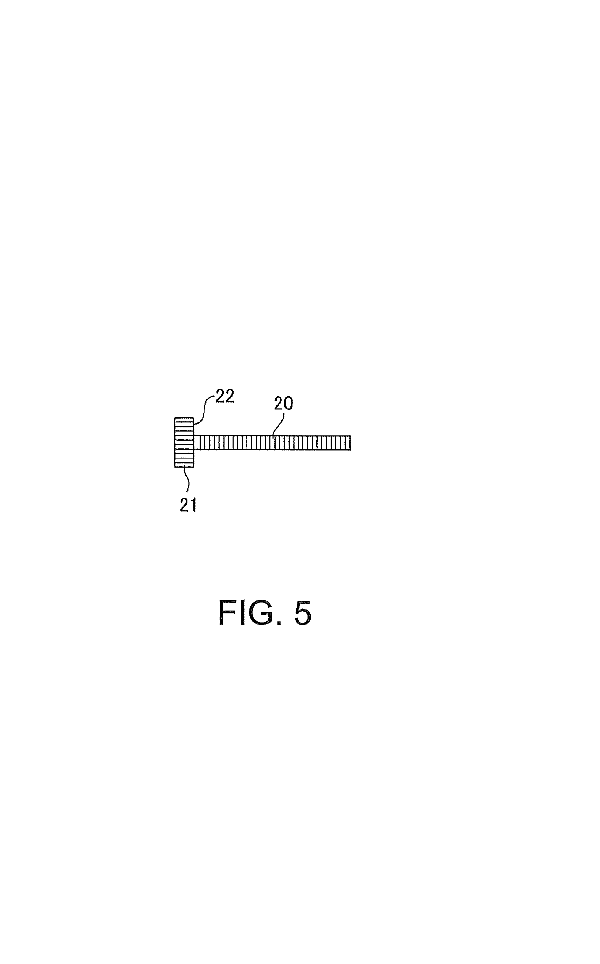

Then, as illustrated in FIG. 4, an approximate-T-shaped cutout 11 to which a thin-plate-use male screw 20 (see FIG. 5) having a head portion 21 can be inserted from the thickness direction is arranged at the other end side (the side to be anchored to the support pillar of the detector W) of the thin plate 10 on the plane extending in the width direction and the length direction.

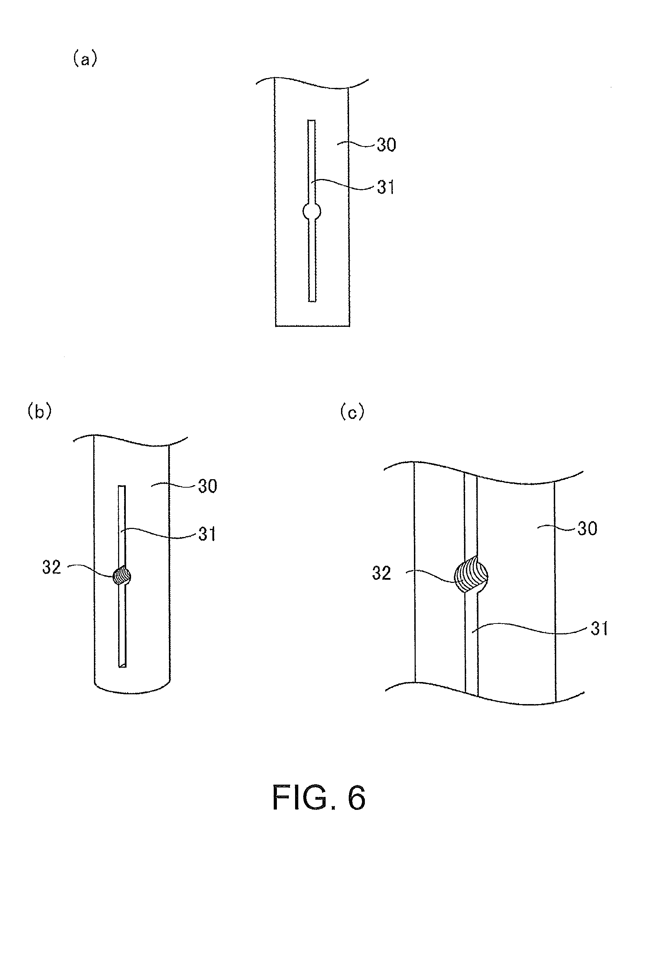

Further, as illustrated in FIG. 6, a slit 31 longer than the width of the thin plate 10 is formed at the support pillar 30 of the detector W in the longitudinal direction. A female thread 32 to which the thin-plate-use male screw 20 can be screwed is formed in the slit 31.

Then, the thin plate 10 is inserted to the slit 31 of the support pillar 30. FIG. 6(a) is a plane view of the support pillar 30 on a plane extending in the width direction and the thickness direction of the thin plate 10, assuming that the thin plate 10 is inserted to the support pillar 30, FIG. 6(b) is a perspective view thereof, and FIG. 6 (c) is an enlarged view of a main part of FIG. 6 (b) (an enlarged view of a part of the female thread 32).

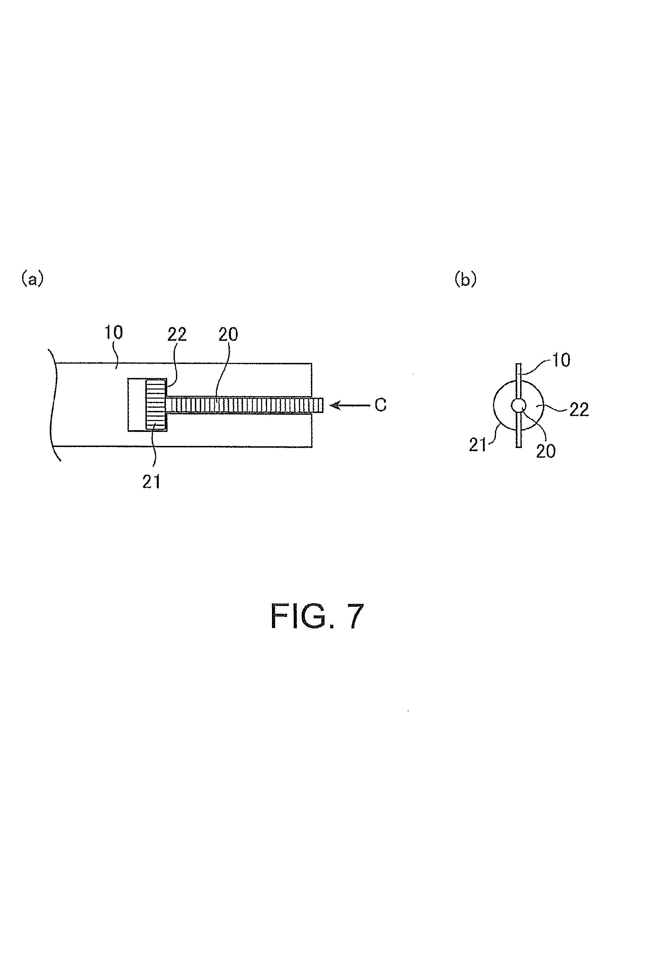

FIG. 7 is a view illustrating a state that the thin-plate-use male screw 20 illustrated in FIG. 5 is inserted from the thickness direction to the approximate-T-shaped cutout 11 of the thin plate 10 illustrated in FIG. 4(a). FIG. 7 (a) is a plane view on the plane extending in the width direction and the length direction. FIG. 7 (b) is a side view viewing FIG. 7 (a) from a direction of arrow C. As illustrated in the drawings, the diameter of a shaft portion of the thin-plate-use male screw 20 is larger than the thickness of the thin plate 10 and the diameter of the head portion 21 of the thin-plate-use male screw 20 is significantly larger than the thickness of the thin plate 10. Accordingly, it is possible to turn the head portion 21 even in a state that the thin-plate-use male screw 20 is inserted to the approximate-T-shaped cutout 11 of the thin plate 10.

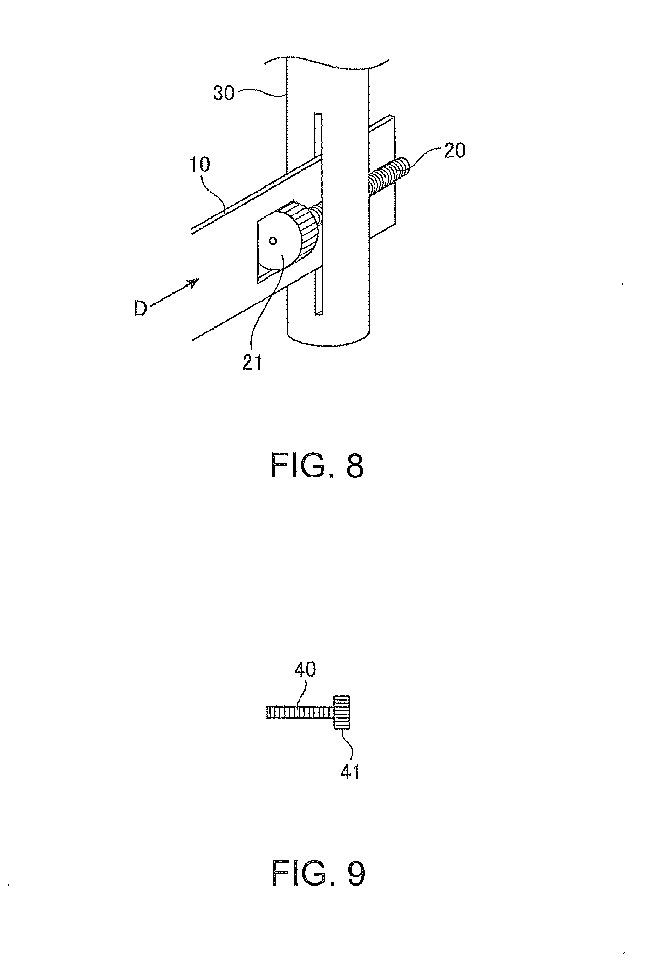

FIG. 8 is a view illustrating a state that the thin plate 10 with the thin-plate-use male screw 20 inserted to the approximate-T-shaped cutout 11 of the thin plate 10 as illustrated in FIG. 7 is inserted to the slit 31 of the support pillar 30 and anchored thereto. As illustrated in the drawing, the thin plate 10 is inserted to the slit 31 of the support pillar 30, and the thin-plate-use male screw 20 is screwed to the female thread 32 in the slit 31 by turning the head portion 21 of the thin-plate-use male screw 20 so that a seating face 22 of the thin-plate-use male screw 20 pushes a thickness section of the approximate-T-shaped cutout 11 in a direction toward the support pillar 30 (in a direction of arrow D in FIG. 8). Thus, the thin plate 10 can be anchored to the support pillar 30 to be positionally adjustable.

Thus, owing to that the female thread 32 to which the thin-plate-use male screw 20 is screwed is formed in the slit 31, the position of the thin plate 10 can be finely adjusted without using a separate part such as a nut. Accordingly, even in a case that positional adjustment is required to be performed at a site where the oil film detection device is installed, the positional adjustment of the thin plate 10 and the light-receiving portion 4 can be easily performed without a fear of losing a part such as a nut and without using a tool and the like.

In the second embodiment, the slit 31 of the support pillar 30 is described as a slit whose both ends in the longitudinal direction are not opened. However, it is also possible to adopt a slit having an open end at an end of the support pillar (lower part in FIG. 6(a)).

Further, as illustrated in FIG. 3, in the oil film detection device according to the second embodiment, the light-receiving portion 4 is anchored by the three thin plates 10 and three support pillars 30 are arranged corresponding to the respective thin plates 10. However, as long as the light-receiving portion 4 can be supported and positional adjustment of the light-receiving portion 4 can be performed, any number of sets of the thin plates 10 and the support pillars 30 may be adopted. Here, to prevent laser light and reflection light from being blocked to the extent possible, it is preferable to adopt less number thereof. In addition, to perform positional adjustment of the light-receiving portion 4 while performing supporting thereof, it is the most preferable to arrange three sets of the thin plates 10 and the support pillars 30 as illustrated in FIG. 3.

As described above, according to the oil film detection device using the thin plate anchoring method in the second embodiment of the present invention, the thin plate can be easily anchored while performing positional adjustment using the thin-plate-use male screw for anchoring the thin plate to the support pillar.

### Third embodiment

Similarly to the second embodiment, a third embodiment of the present invention will be described as exemplifying a case that a thin plate anchoring method for anchoring a thin plate to a support pillar is adopted to the oil film detection device illustrated in FIGs. 1 and 3.

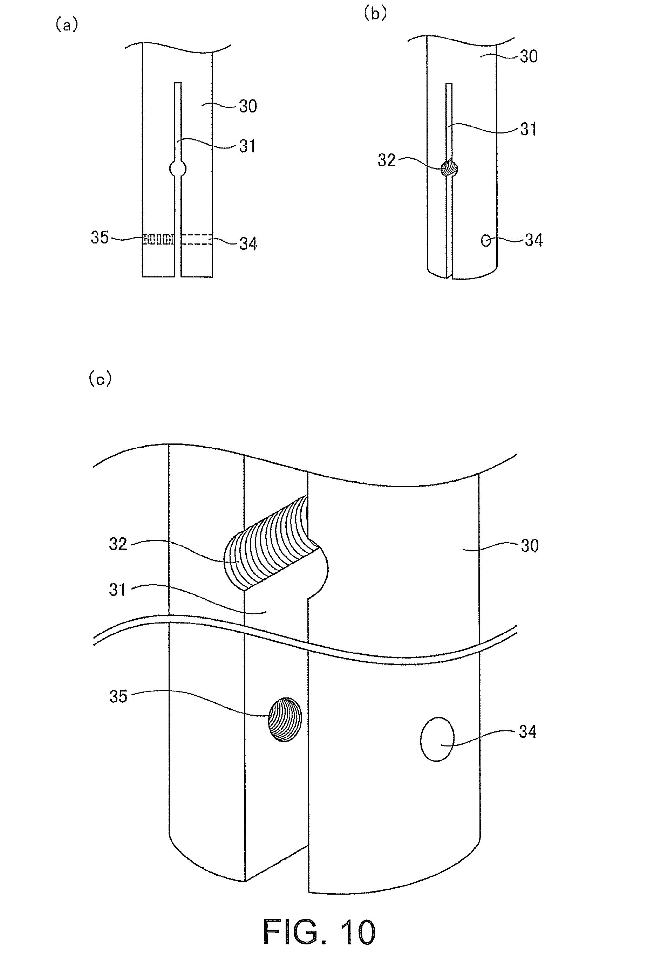

In the third embodiment of the present invention, the thin plate 10 and the thin-plate-use male screw 20 are the same as those illustrated in FIGs. 4, 5, and 7 in the second embodiment. Here, a support pillar 30 illustrated in FIG. 10 is used instead of the support pillar 30 illustrated in FIG. 6.

Here, for anchoring the thin plate 10 to the support pillar 30 more reliably, an anchoring screw 40 illustrated in FIG. 9 is adopted. As illustrated in FIG. 10, in addition to the slit 31 and the female thread 32 to which the thin-plate-use male screw 20 is screwed, the support pillar 30 includes a through-hole 34 for the anchoring screw and a female thread 35 for the anchoring screw which are located at positions where the slit 31 is formed and interference with the thin plate 10 is not caused when the thin plate 10 is inserted to the slit 31.

Then, the thin plate 10 is inserted to the slit 31 of the support pillar 30. FIG. 10 (a) is a plane view of the support pillar 30 on a plane extending in the width direction and the thickness direction of the thin plate 10, assuming that the thin plate 10 is inserted thereto, FIG. 10 (b) is a perspective view thereof, and FIG. 10 (c) is an enlarged view of a main part of FIG. 10 (b) (an enlarged view of a part of the female thread 32 and an enlarged view of a part of the through-hole 34 for the anchoring screw and the female thread 35 for the anchoring screw).

Here, the third embodiment is described with an example that the slit is formed with an end of the support pillar 30 (lower part in FIG. 10(a)) opened. However, similarly to the second embodiment, it is also possible to adopt a slit without having an open end.

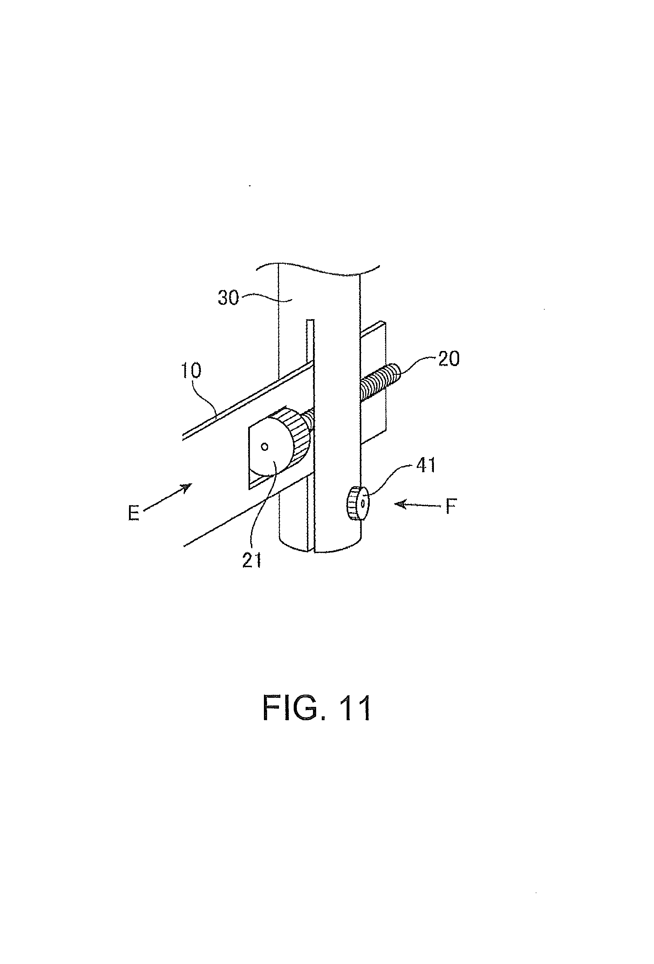

FIG. 11 is a view illustrating a state that the thin plate 10 with the thin-plate-use male screw 20 inserted to the approximate-T-shaped cutout 11 of the thin plate 10 as illustrated in FIG. 7 is inserted to the slit 31 of the support pillar 30 illustrated in FIG. 10 and anchored thereto. As illustrated in the drawing, the thin plate 10 is inserted to the slit 31 of the support pillar 30, and the thin-plate-use male screw 20 is screwed to the female thread 32 in the slit 31 by turning the head portion 21 of the thin-plate-use male screw 20 so that a seating face 22 of the thin-plate-use male screw 20 pushes a thickness section of the approximate-T-shaped cutout 11 in a direction toward the support pillar 30 (in a direction of arrow E in FIG. 11). Thus, the thin plate 10 can be anchored to the support pillar 30 to be positionally adjustable.

Further, the anchoring screw 40 illustrated in FIG. 9 is inserted (in a direction of arrow F in FIG. 11) to the through-hole 34 for the anchoring screw arranged at the support pillar 30, and the anchoring screw 40 is screwed to the female thread 35 for the anchoring screw through the through-hole 34 for the anchoring screw by turning a head portion 41 of the anchoring screw 40 so that a seating face of the anchoring screw 40 pushes the support pillar 30. Thus, the thin plate 10 after positional adjustment is reliably anchored to the support pillar 30.

Here, the through-hole 34 for the anchoring screw and the female thread 35 for the anchoring screw are separated by the slit 31 as illustrated in FIG. 10 as having size to allow the anchoring screw 40 to be inserted thereto. The through-hole 34 for the anchoring screw is a simple hole without a female thread formed therein. Therefore, when the anchoring screw 40 is inserted to the through-hole 34 for the anchoring screw, the insertion can be easily performed until the leading end of the anchoring screw 40 exceeds the slit 31. Thereafter, the anchoring screw 40 is screwed to the female thread 35 for the anchoring screw through the through-hole 34 for the anchoring screw by turning the head portion 41 of the anchoring screw 40, and then, the head portion 41 is turned to a position where the seating face of the anchoring screw 40 pushes the support pillar 30. Thus, the anchoring can be reliably performed.

In a case that the thin-plate anchoring method is applied to the oil film detection device illustrated in FIGs. 1 and 3, for performing positional fine adjustment of the light-receiving portion 4 of the oil film detection device, positional adjustment of the thin plate 10 is performed by turning the thin-plate-use male screw 20 respectively at three positions of the support pillars 30 arranged at the detector W. After the position of the light-receiving portion 4 is determined owing to the positional adjustment of the three thin plates 10, the anchoring screws 40 respectively at the three positions are rotated so that the thin plates 10 can be anchored to the corresponding support pillars 30 more reliably. As a result, the light-receiving portion 4 can be reliably anchored at the positionally adjusted position.

Thus, owing to that the female thread 35 for the anchoring screw is formed at the support pillar 30, the thin plate 10 can be reliably anchored without using a separate part such as a nut. Accordingly, even in a case that positional adjustment is required to be performed at a site where the oil film detection device is installed, the positional adjustment and reliable anchoring of the thin plate 10 and the light-receiving portion 4 can be easily performed without a fear of losing a part such as a nut.

As described above, according to the oil film detection device using the thin plate anchoring method in the third embodiment of the present invention, the thin plate anchored to the support pillar with positional adjustment can be anchored more reliably, in addition to the effect of the second embodiment.

In the description of the second and third embodiments, the support pillar 30 is formed as a cylindrical support pillar. However, it is obviously possible to adopt a support pillar having a different shape such as a square pillar and a hexagonal pillar.

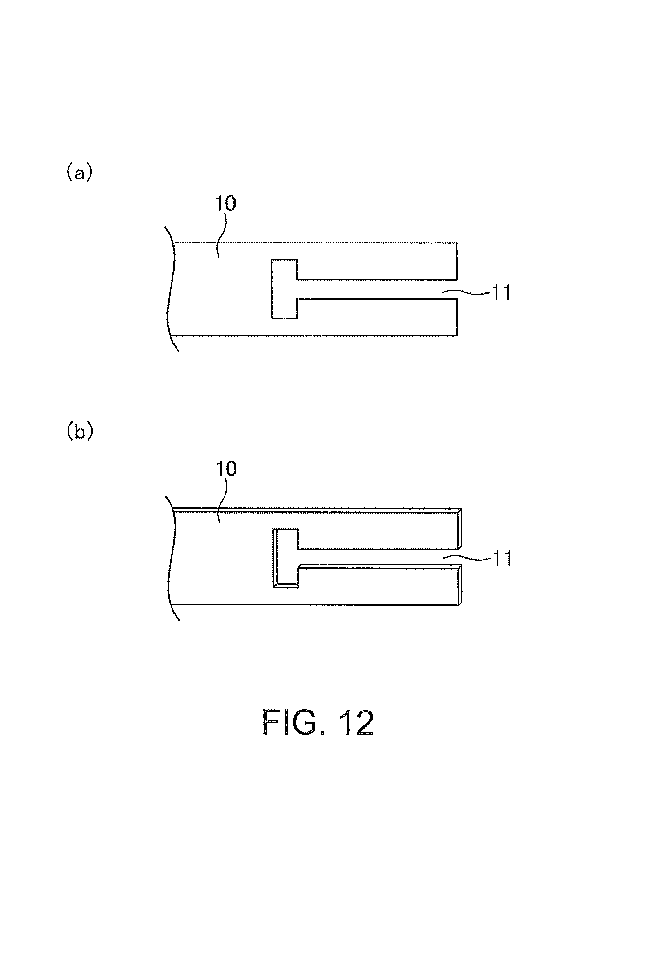

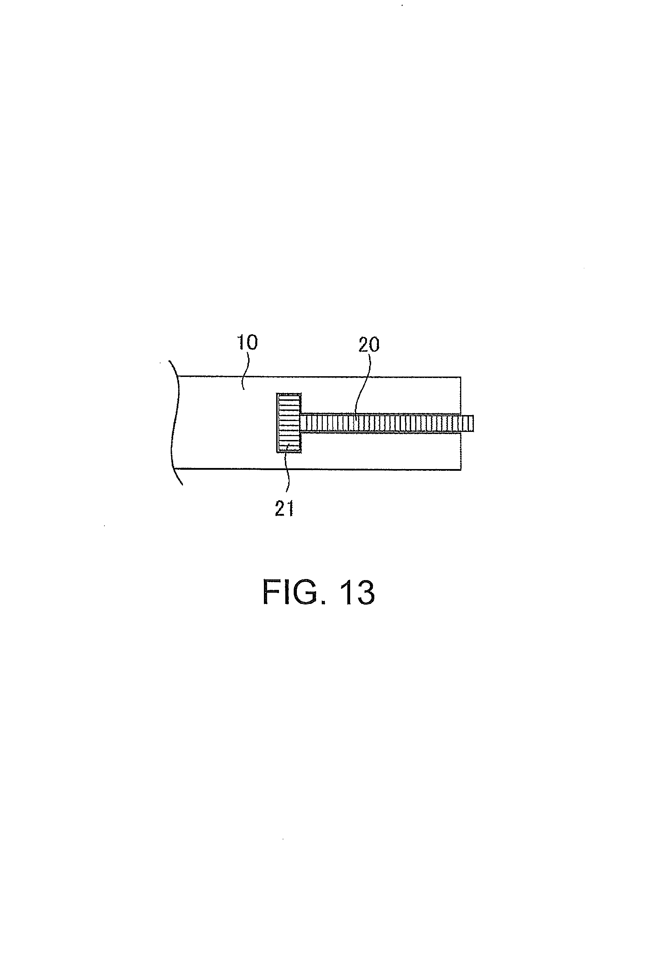

Further, in the description of the approximate-T-shaped cutout 11 formed at the thin plate 10 in the second and third embodiments, a section to which the head portion 21 of the thin-plate-use male screw 20 is inserted has a larger length than the head portion 21 in the length direction, as illustrated in FIG. 4. However, as illustrated in FIGs. 12 and 13, the approximate-T-shaped cutout 11 may be formed to have approximately the same size as the head portion 21 of the thin-plate-use male screw 20.

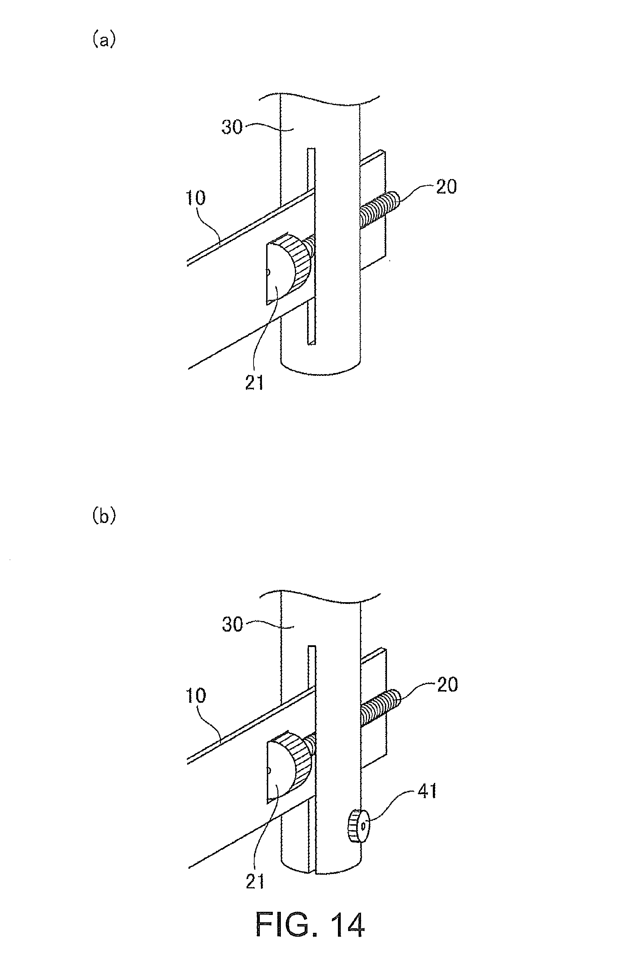

FIGs. 14(a) and 14(b) illustrate a state that the thin plate 10 is anchored after being inserted to the slit 31 of the support pillar 30 in the above case of the second and third embodiments (as corresponding to FIGs. 8 and 11).

Here, when the head portion 21 of the thin-plate-use male screw 20 is turned, the seating face 22 of the thin-plate-use male screw 20 becomes immediately in a state to push a thickness section of the approximate-T-shaped cutout 11 in the direction toward the support pillar 30. Accordingly, response at the time of positional adjustment is further improved.

Within the scope of the present invention, it is possible to adopt flexible combination of the respective embodiments, modification of an arbitrary structural element in the respective embodiments, or elimination of an arbitrary structural element in the respective embodiments.

### EXPLANATION OF REFERENCES

- W, 100: Detector
- 1, 110: Light-source and scanning portion
- 2, 120: Concave mirror
- 3: Through-hole
- 4, 140: Light-receiving portion
- 5, 150: Accommodating portion
- 6, 160: Window
- 7: Emitting window
- 9a, 9b, 190: Water surface (Detection target surface)
- 10: Thin plate
- 11: Approximate-T-shaped cutout
- 20: Thin-plate-use male screw
- 21: Head portion of thin-plate-use male screw 20
- 22: Seating face of thin-plate-use male screw 20
- 30: Support pillar
- 31: Slit of support pillar 30
- 32: Female thread
- 34: Through-hole for anchoring screw
- 35: Female thread for anchoring screw
- 40: Anchoring screw
- 41: Head portion of anchoring screw 40

## Claims

1. An oil film detection device, comprising, in a detector which is arranged approximately in parallel to a detection target surface extended in the horizontal direction, an accommodating portion which accommodates at least a light source, a concave mirror, and a light-receiving portion, and a window which closes the accommodating portion in a watertight manner, for detecting an oil film existing on the detection target surface owing to that light is radiated from the light source toward the detection target surface, and reflection light from the detection target surface is collected by the concave mirror and received by the light-receiving portion,
the light source being arranged above the concave mirror and emitting light approximately in the vertical direction,
the concave mirror being arranged so that an optical axis of the concave mirror is perpendicular to the horizontal direction and having a through-hole, at a position deviated from the optical axis of the concave mirror, through which emitted light from the light source passes, and
the light-receiving portion being arranged at a focal position on the optical axis of the concave mirror.

2. The oil film detection device according to claim 1,
wherein the window is arranged in parallel to a horizontal section of the detector, and
an emitting window through which the emitted light penetrates is arranged at a part of the window as being inclined from the horizontal surface by a predetermined angle.

3. The oil film detection device according to claim 1 or claim 2,
wherein the light-receiving portion is held by at least two thin plates, each thin plate having a width in a direction of an optical axis of radiation from the light source, and a length larger than the width and a thickness smaller than the width respectively in a direction perpendicular to the optical axis,
an approximate-T-shaped cutout capable of allowing a thin-plate-use male screw having a head portion to be inserted thereto from a direction of the thickness is formed at each thin plate on a plane extending in a direction of the width and a direction of the length,
the detector includes support pillars corresponding to the respective thin plates,
the support pillar is formed with a slit longer than the width of the thin plate in the longitudinal direction and a female thread formed in the slit to allow the thin-plate-use male screw to be screwed thereto, and
the thin plates are anchored to the corresponding support pillars in a positionally adjustable manner owing to that each thin plate is inserted to the slit of the corresponding support pillar and the thin-plate-use male screw is screwed to the female thread in the slit so that a seating face thereof pushes a thickness section of the approximate-T-shaped cutout in a direction toward the support pillar.

4. The oil film detection device according to claim 3,
wherein each support pillar is formed with a through-hole for an anchoring screw and a female thread for the anchoring screw at positions where the slit are formed and interference with the thin plate is not caused, and
the anchoring screw is screwed to the female thread for the anchoring screw through the through-hole for the anchoring screw.
